# EUROPÄISCHE PATENTSCHRIFT

(11) **EP 2 318 024 B1**
(45) Veröffentlichungstag und Bekanntmachung des Hinweises auf die Patenterteilung: **08.05.2019**
(21) Anmeldenummer: 09777023.4
(22) Anmeldetag: 08.07.2009
(51) Int. Cl.: A61K 36/23, A61K 31/728, A61P 17/00, A61Q 19/06

(54) **WIRKSTOFFKOMBINATIONEN AUS ANISFRUCHTEXTRAKT UND HYALURONSÄURE**
ACTIVE SUBSTANCE COMBINATIONS CONSISTING OF AN ANIS FRUIT EXTRACT AND HYALURONIC ACID
COMBINAISONS DE PRINCIPES ACTIFS CONSTITUÉES D'EXTRAIT DE FRUITS D'ANIS ET D'ACIDE HYALURONIQUE

(30) Priorität: 22.07.2008 DE 102008034265
(43) Veröffentlichungstag der Anmeldung: 11.05.2011
(73) Patentinhaber: Beiersdorf AG, 20253 Hamburg (DE)
(72) Erfinder: ESPEL, Anja, 22041 Hamburg (DE); SCHÄFER, Jessica, 22113 Oststeinbek (DE); FEY, Sven, 22397 Hamburg (DE); BREITENBACH, Ute, 22299 Hamburg (DE); KNOTT, Anja, 22527 Hamburg (DE); MUMMERT, Christoper, 29553 Bienenbüttel (DE); SCHERNER, Cathrin, 22844 Norderstedt (DE)
(74) Vertreter: Wilke, Jochen
(86) Internationale Anmeldenummer: PCT/EP2009/004924
(87) Internationale Veröffentlichungsnummer: WO 2010/009809

(56) Entgegenhaltungen:
- WO-A1-00/38647
- WO-A1-2006/128584

## Beschreibung

Die vorliegende Erfindung betrifft Wirkstoffkombinationen aus einer wirksamen Menge an Anisfruchtextrakt und Hyaluronsäure sowie kosmetische Zubereitungen, solche Wirkstoffkombinationen enthaltend.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie der unter a), e) und g) aufgeführten Phänomene.

Produkte zur Pflege erschlaffter, insbesondere gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Daüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Oftmals wird mit der erschlafften Haut auch eine Begleiterscheinung der Übergewichtigkeit und/oder der damit häufig einhergehenden sogenannten Cellullite verbunden.

Das Körperbewußtsein der Verbraucher ist in den vergangenen Jahren deutlich gestiegen. Dabei werden neben reinigenden und pflegenden Anwendungen auch zunehmend Maßnahmen ergriffen, um die Körpersilhouette zu verbessern. Die Cellulite - ein weit verbreitetes Phänomen - nimmt dabei eine zentrale Stellung ein. Das sichtbare Bild der Cellulite beruht auf einer Zunahme von Fettpolstern in der Subcutis (Unterhautfettgewebe), einer Bindegewebsschwäche sowie einer Minderung der Durchströmungsverhältnisse in den Blut- und Lymphbahnen. Die Ursache ist somit eine zum Teil anlagebedingte Schwächung des Bindegewebes mit gleichzeitigem Auftreten von vergrößerten Fettzellkammern infolge von Übergewicht, unausgewogener Ernährung, Bewegungsmangel. Die Entstehung der Cellulite kann ferner auf eine erhöhte Durchlässigkeit der Haargefäßwände zurückgeführt werden, die das Eindringen von Wasser ins Bindegewebe erlaubt.

Daneben kann an den betroffenen Hautbereichen ein lokaler Mangel an Testosteron bestehen. Jedenfalls ist Cellulite eine Erscheinung die fast niemals bei Männern zu beobachten ist.

Aufgabe der vorliegenden Erfindung war es somit, Wege zu finden, die die Nachteile des Standes der Technik vermeiden. Insbesondere war es eine Aufgabe der vorliegenden Erfindung, Zubereitungen zur Verfügung zu stellen, die eine vorteilhafte Straffung erschlaffter Haut bewirken können.

Eine weitere Aufgabe der vorliegenden Erfindung war es, hautpflegende Zubereitungen und Zubereitungen zur Pflege der Haut zur Verfügung zu stellen, wobei die Zubereitungen das Körperäußere pflegen und verschönern sollten. Dabei sollten die Zubereitungen insbesondere auch die Hautfeuchtigkeit verbessern.

Es hat sich überraschenderweise herausgestellt, daß eine kosmetische Zubereitung, dadurch gekennzeichnet, daß sie eine kosmetisch wirksame Menge an Anisfruchtextrakt und Hyaluronsäure gemäß Anspruch 1 enthält, den Nachteilen des Standes der Technik abhilft.

Die Verwendung erfindungsgemäßer Zubereitungen mit einem kosmetisch wirksamen Gehalt an Anisfruchtextrakt und Hyaluronsäure - z. B. in Form der genannten Beispiele - führt in überraschender Weise zu einer erheblichen Verbesserung des Aussehens der Haut, insbesondere werden durch Anwenden erfindungsgemäßer Zubereitungen
- die Barriereeigenschaften der Haut erhalten oder wiederhergestellt,
- besser der Hautaustrocknung entgegengewirkt sowie
- die Haut besser vor Umwelteinflüssen geschützt.

Durch die Verwendung erfindungsgemäßer Zubereitungen wird ferner die Hautfeuchtigkeit und die Tiefenfeuchtigkeit der Haut gesteigert, wobei diese hautbefeuchtende Pflege-Wirkung und der Schutz vor Feuchtigkeitsverlust über mehrere Stunden bzw. sogar über den ganzen Tag oder noch länger anhält.

Erfindungsgemäß ist daher auch die Verwendung kosmetischer Zubereitungen, die eine kosmetisch wirksame Menge an Anisfruchtextrakt und Hyaluronsäure enthalten, zur Steigerung der Hautfeuchtigkeit bzw. zur Befeuchtung der Haut.

Die erfindungsgemäßen Zubereitungen eignen sich darüber hinaus hervorragend zur Pflege sensibler Haut.

Es war ferner überraschend, dass durch Anwenden erfindungsgemäßer Zubereitungen die Elastizität der Haut, der Haare und/oder der Nägel und daher Cellulite bzw. das Erscheinungsbild der sog. "Orangenhaut" verbessert wird.

Bevorzugt enthalten kosmetische oder dermatologische Zubereitungen gemäß der Erfindung 0,001 bis 30 Gew.-%, bevorzugt 0,1 bis 15 Gew.-%, besonders bevorzugt 0,5 bis 5 Gew.-% an Anisfruchtextrakt und Hyaluronsäure, jeweils bezogen auf das Gesamtgewicht der Zubereitung.

Es ist erfindungsgemäß insbesondere vorteilhaft, einen wäßrigen Frucht-Extrakt aus Anis (INCI: Pimpinella anis fruit extract), der reich an anorganischen Mineralien wie Natrium- und Magnesium-Ionen, besonders an Kalium-Ionen ist und mit Butylenglycol 0,36% und Parabenen 0,14% konserviert ist und durch enzymatische Hydrolyse in Wasser solubilisierter Anis-Früchte erhältlich ist, wobei das Verhältnis von Rohmaterial zu Extrakt etwa 1 : 2 beträgt und unter dem Handelsnamen Bioxilift ® bei der Gesellschaft SILAB, (Brive Cedex, France) erhältlich ist.

Bei Anisfruchtextrakt (Bioxylift ®, Fa. SILAB) handelt es sich um einen wäßrigen Frucht-Extrakt aus Anis.

Der Extrakt weist eine bernsteinartige Farbe auf und hat einen charakteristischen Anis-Geruch. Er ist reich an anorganischen Mineralien wie Natrium- und Magnesiumionen, besonders aber an Kaliumionen.

Die spezifische Zusammensetzung beträgt:

| | |
|---|---|
| Trockenmasse: | 40 - 60 g/l |
| Mineralasche | 11 - 18 g/l |
| Gesamtprotein | 12 - 20 g/l |
| pH | 4,5 - 5,5 |

Der Extrakt ist unkonserviert oder auch konserviert herstell- und lagerbar. Seine Herstellung wird in der Internationalen Patentanmeldung WO 02/102347 beschrieben.

Eine mögliche Konservierung ist beispielsweise mit Butylenglycol 0,36% und Parabenen 0,14% und ist in Ethanol bis zu einem Verhältnis von 40/60 Ethanol/Wasser (v/v) löslich. Bei der Herstellung werden die Anis-Früchte in Wasser solubilisiert und enzymatisch hydrolysiert. Das Verhältnis von Rohmaterial zu Extrakt beträgt 1 : 2. Lösliche und unlösliche Phasen werden getrennt und anschließend filtriert bzw. danach steril filtriert.

Hyaluronsäure ist ein Polysaccharidderivat, welches durch folgendes Strukturelement gekennzeichnet ist:

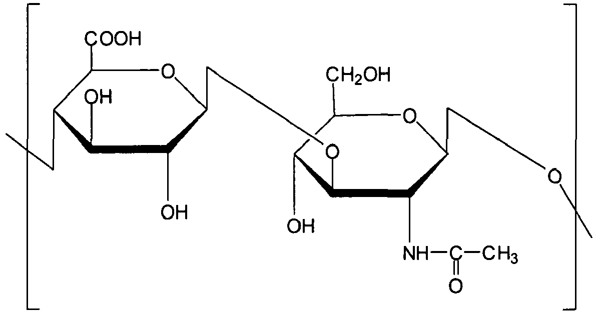

Hyaluronsäure ist ein Glycosaminoglycan, das im Bindegewebe, im Glaskörper, in der Nabelschnur und in der Synovialflüssigkeit der Gelenke vorkommt. Hyaluronsäure ist in besonders hohem Maße in frühen Embryonalstadien vorhanden und wirkt hier an Zellwanderungen mit, indem sie durch ihre lockere Struktur und einen hohen Wassergehalt die sie auf ihrer Oberfläche exprimierenden Zellen gegeneinander abschirmt und so deren freie Beweglichkeit ermöglicht. Hyaluronsäure ist an der Wundheilung beteiligt

Hyaluronsäure ist eine hochmolekulare Verbindung mit Molekulargewichten zwischen 40000 und mehreren Millionen. Eingesetzt wird die kurzkettige Hyaluronsäure mit einem mittleren Molmassenmaximum zwischen 40.000 und 60.000 g/mol, insbesondere etwa von 52.000 g/mol, also 52kDa, Es ist erfindungsgemäß gleichermaßen vorteilhaft, wenn die Hyaluronsäure in protonierter Form vorliegt, als Salz, als teilprotoniertes Salz, in Lösung dissoziiert oder teildissoziiert

Erfindungsgemäß beträgt die Konzentration an Hyaluronsäure bezogen auf das Gesamtgewicht der Zubereitung vorteilhaft 0,001 bis 10 Gewichts-%, bevorzugt von 0,05 bis 5 Gewichts-% und ganz besonders bevorzugt von 0,01 bis 2 Gewichts-%, jeweils bezogen auf das Gesamtgewicht der Zubereitung.

Vorteilhaft werden Gewichtsverhältnisse von Anisfruchtextrakt und Hyaluronsäure aus den Bereichen 1 : 10 bis 10 : 1, bevorzugt von 1 :5 bis 5 : 1,besonders bevorzugt von 1 : 3,33 bis 3,3 : 1, gewählt.

Die erfindungsgemäßen Wirkstoffkombinationen lassen sich problemlos üblichen kosmetischen und/oder dermatologischen Zubereitungen, wie Lichtschutzzubereitungen, Hautpflegezubereitungen, Antifalten-Zubereitungen, aber auch anderen Zubereitungen, beispielsweise pharmazeutischen Zubereitungen einverleiben.

Die kosmetischen oder dermatologischen Zubereitungen können erfindungsgemäß wie üblich zusammengesetzt sein und zur Behandlung, der Pflege und der Reinigung der Haut und/oder der Haare und als Schminkprodukt in der dekorativen Kosmetik dienen.

Es ist auch möglich und vorteilhaft im Sinne der vorliegenden Erfindung, die erfindungsgemäßen Wirkstoffkombinationen in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Siliconderivate.

Die Menge an Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Siliconöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, Gewichtsprozente, auf das Gewicht und die Gesamtmenge bzw. auf das Gesamtgewicht der Zubereitungen bezogen.

| **Beispiel 1** | |
|---|---|
| **I** | **Gew-%** |
| 1,2-Hexandiol | 1,00 |
| Behenylalkohol | 2,00 |
| Caprylsäure/Caprinsäuretriglyceride | 6,00 |
| Carbomer | 0,30 |
| Cetylalkohol | 3,00 |
| Dicaprylylether | 5,00 |
| Ethylhexylglycerin | 0,00 |
| Glycerin | 0,50 |
| Glycerylstearatcitrat | 1,50 |
| Methylpropandiol | 4,00 |
| Phenoxyethanol | 0,50 |
| Polymethylsilsesquioxan | 2,00 |
| Natriumhyaluronat | 0,10 |
| Stearylalkohol | 4,00 |
| Titanium Dioxide + Trimethoxycaprylylsilan | 2,00 |
| Wasser + Pimpinella Anisum (Anisfruchtextrakt) | 3,50 |
| Natriumhydroxid | 0,60 |
| Wasser | Ad 100,00 |

| **Beispiel 2** | |
|---|---|
| **I** | **Gew-%** |
| Acrylat/C10-30 Alkylacrylat-Crosspolymer | 0,10 |
| Butylenglycoldicaprylat/dicaprat | 3,50 |
| Butyrospermum Parkii (Shea Butter) | 6,00 |
| C12-15 Alkylbenzoat | 1,50 |
| Caprylsäure/Caprinsäuretriglyceride | 1,00 |
| Cetylstearylalkohol | 3,00 |
| Cetylalkohol | 4,00 |
| Chondrus Crispus (Carrageenan) | 0,50 |
| Diethylaminohdroxybenzoylhexylbenzoat | 3,00 |
| Dimethicon | 0,50 |
| Ethylhexylsalicylat | 4,00 |
| Ethylhexylglycerin | 0,50 |
| Glycerin | 10,00 |
| Glycerylstearat SE | 2,00 |
| Methylparaben | 0,10 |
| Methylpropanediol | 2,00 |
| Microcrystallines Wachs + Mineralöl | 1,50 |
| Phenoxyethanol | 0,30 |
| Phenylbenzimidazolsulfonsäure | 1,50 |
| Red 40 | 0,10 |
| Natriumhyaluronat | 0,10 |
| TiO₂ + Al-Hydroxid + Dimethicone/Methicone Copolymer | 1,00 |
| Tocopherylacetat | 0,50 |
| Na₃HEDTA | 1,00 |
| Wasser + Pimpinella Anisum (Anisfruchtextrakt) | 4,00 |
| Water + NaOH | 0,70 |
| Wasser | Ad 100,00 |

| **Beispiel 3** | |
|---|---|
| **I** | **Gew-%** |
| Acrylat/C10-30 Alkylacrylat-Crosspolymer | 0,15 |
| Butylenglycoldicaprylat/dicaprat | 8,00 |
| Butyrospermum Parkii (Shea Butter) | 3,00 |
| C12-15 Alkylbenzoat | 1,00 |
| Caprylsäure/Caprinsäuretriglyceride | 6,00 |
| Caprylylglycol | 0,25 |
| Cetylstearylalkohol | 5,00 |
| Chondrus Crispus (Carrageenan) | 0,25 |
| Dimethicon | 1,00 |
| Ethylhexylglycerin | 0,50 |
| Glycerin | 10,00 |
| Glycerylstearat | 2,00 |
| Methylparaben | 0,20 |
| Panthenol | 1,50 |
| Phenoxyethanol | 0,50 |
| Red 40 | 0,03 |
| Natriumhyaluronat | 0,20 |
| Natriumstearoylglutamat | 0,30 |
| Sunset Yellow | 1,00 |
| Tocopheryl Acetat | 0,50 |
| Trisodium EDTA | 1,00 |
| Wasser + Pimpinella Anisum (Anisfruchtextrakt) | 5,00 |
| Natriumhydroxid | 0,08 |
| Wasser | Ad 100,00 |

## Patentansprüche

1. Wirkstoffkombinationen aus einer wirksamen Menge an einem wässrigen Anisfruchtextrakt und Hyaluronsäure, **dadurch gekennzeichnet, dass** die Hyaluronsäure aus der Gruppe der Hyaluronsäuren mit Molekulargewichten von 40.000 bis 60.000 g/mol gewählt wird, insbesondere etwa. 52.000 g/mol.

2. Kosmetische Zubereitungen, enthaltend Wirkstoffkombinationen nach Anspruch 1.

3. Zubereitung nach Anspruch 2, **dadurch gekennzeichnet, dass** die Konzentration an Anisfruchtextrakt von 0,0001 bis 30 Gew.-% gewählt wird.

4. Zubereitung nach einem der vorhergehenden Ansprüche, **dadurch gekennzeichnet, dass** die Konzentration an Anisfruchtextrakt von 0,5 bis 10 Gew.-% gewählt wird.

5. Zubereitung nach einem der vorhergehenden Ansprüche, **dadurch gekennzeichnet, dass** die Konzentration an Hyaluronsäure von 0,5 bis 10 Gew.-% gewählt wird.

6. Wirkstoffkombinationen oder Zubereitungen nach einem der vorgehenden Ansprüche, **dadurch gekennzeichnet, dass** Gewichtsverhältnisse von Anisfruchtextrakt und Hyaluronsäure aus den Bereichen 1 : 10 bis 10 : 1, bevorzugt von 1 :5 bis 5 : 1, besonders bevorzugt von 1 : 3,33 bis 3,3 : 1, gewählt.

## Claims

1. Active ingredient combinations of an effective amount of an aqueous anise fruit extract and hyaluronic acid, **characterized in that** the hyaluronic acid is selected from the group of hyaluronic acids having molecular weights of 40 000 to 60 000 g/mol, especially approximately 52 000 g/mol.

2. Cosmetic preparations comprising active ingredient combinations according to Claim 1.

3. Preparation according to Claim 2, **characterized in that** the concentration of anise fruit extract selected is from 0.0001 to 30% by weight.

4. Preparation according to any of the preceding claims, **characterized in that** the concentration of anise fruit extract selected is from 0.5 to 10% by weight.

5. Preparation according to any of the preceding claims, **characterized in that** the concentration of hyaluronic acid selected is from 0.5 to 10% by weight.

6. Active ingredient combinations or preparations according to any of the preceding claims, **characterized in that** ratios by weight of anise fruit extract and hyaluronic acid are selected from the ranges from 1:10 to 10:1, preferably from 1:5 to 5:1, particularly preferably from 1:3.33 to 3.3:1.

## Revendications

1. Combinaisons d'agents actifs constituées par une quantité efficace d'un extrait aqueux d'anis et d'acide hyaluronique, **caractérisées en ce que** l'acide hyaluronique est choisi dans le groupe des acides hyaluroniques ayant des poids moléculaires de 40 000 à 60 000 g/mol, notamment d'environ 52 000 g/mol.

2. Préparations cosmétiques, contenant des combinaisons d'agents actifs selon la revendication 1.

3. Préparation selon la revendication 2, **caractérisée en ce que** la concentration en extrait d'anis est choisie de 0,0001 à 30 % en poids.

4. Préparation selon l'une quelconque des revendications précédentes, **caractérisée en ce que** la concentration en extrait d'anis est choisie de 0,5 à 10 % en poids.

5. Préparation selon l'une quelconque des revendications précédentes, **caractérisée en ce que** la concentration en acide hyaluronique est choisie de 0,5 à 10 % en poids.

6. Combinaisons d'agents actifs ou préparations selon l'une quelconque des revendications précédentes, **caractérisées en ce qu'**un rapport en poids entre l'extrait d'anis et l'acide hyaluronique dans la plage allant de 1:10 à 10:1, de préférence de 1:5 à 5:1, de manière particulièrement préférée de 1:3,33 à 3,3:1 est choisi.
